(19) Europäisches Patentamt
European Patent Office
Office européen des brevets

(11) Veröffentlichungsnummer: **0 101 901**
**B1**

# (12) EUROPÄISCHE PATENTSCHRIFT

(45) Veröffentlichungstag der Patentschrift: **13.11.85**

(51) Int. Cl.⁴: **C 07 D 249/08**, C 07 D 233/60

(21) Anmeldenummer: **83107179.0**

(22) Anmeldetag: **22.07.83**

## (54) Verfahren zur Herstellung von 1-Azolyl-3,3-dimethyl-1-phenoxy-butan-2-olen.

(30) Priorität: **03.08.82 DE 3228867**

(43) Veröffentlichungstag der Anmeldung:
**07.03.84 Patentblatt 84/10**

(45) Bekanntmachung des Hinweises auf die Patenterteilung:
**13.11.85 Patentblatt 85/46**

(84) Benannte Vertragsstaaten:
**BE CH DE FR GB LI**

(56) Entgegenhaltungen:
**EP - A - 0 001 414**
**EP - A - 0 011 191**

(73) Patentinhaber: **BAYER AG, Konzernverwaltung RP Patentabteilung, D-5090 Leverkusen 1 Bayerwerk (DE)**

(72) Erfinder: **Pflugbeil, Wolf-Dietrich, Dr., Roeberstrasse 8, D-5600 Wuppertal 1 (DE)**
Erfinder: **Krämer, Wolfgang, Dr., Am Eckbusch 39/45, D-5600 Wuppertal 1 (DE)**

EP 0 101 901 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein neues Verfahren zur Herstellung von bekannten fungizid wirksamen 1-Azolyl-3,3-dimethyl-1-phenoxy-butan-2-olen.

Es ist bereits bekanntgeworden, daß man 1-Azolyl-3,3-dimethyl-1-phenoxy-butan-2-ole erhält, wenn man die entsprechenden Keto-Derivate durch die Meerwein-Ponndorf-Verley-Reduktion mit Aluminiumisopropylat in Isopropanol reduziert (vergleiche DE-OS 2 743 767 [LeA 18 424] und DE-OS 2 850 057 [LeA 19 274]).

Die Meerwein-Ponndorf-Verley-Reduktion hat den Nachteil, daß es sich bei diesem Verfahren um eine Gleichgewichtsreaktion handelt. Das bedeutet die Verwendung eines verhältnismäßig großen Überschusses an Reduktionsmittel und die Entfernung einer Reaktionskomponente (z. B. Aceton) aus dem Gleichgewicht, um die gewünschte Gleichgewichtsverschiebung zur Bildung des Reduktionsproduktes zu erreichen.

Es wurde gefunden, daß man die bekannten 1-Azolyl-3,3-dimethyl-1-phenoxy-butan-2-ole der Formel

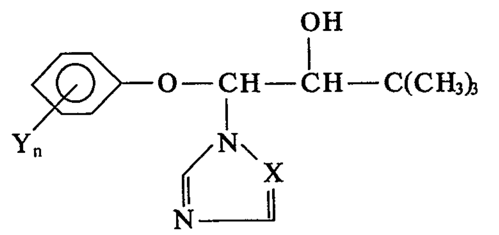

(I)

in welcher

X für ein Stickstoffatom oder die CH-Gruppe steht,
Y für Halogen, Alkyl, Alkoxycarbonyl, Nitro oder gegebenenfalls substituiertes Phenyl steht und
n für ganze Zahlen von 0 bis 3 steht,

erhält, wenn man 1-Azolyl-3,3-dimethyl-1-phenoxy-butan-2-one der Formel

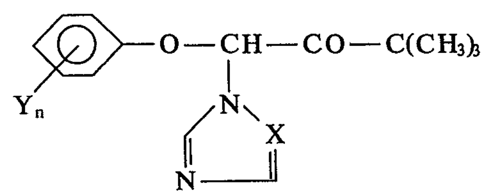

(II)

in welcher
X, Y und n die oben angegebene Bedeutung haben,

in einer Druckreaktion mit sekundären Alkoholaten in Gegenwart eines Verdünnungsmittels umsetzt.

Die Verbindungen der Formel (I) besitzen zwei asymmetrische Kohlenstoffatome; sie können deshalb in den beiden geometrischen Isomeren threo und erythro vorliegen.

Es ist als überraschend zu bezeichnen, daß die Meerwein-Ponndorf-Verley-Reduktion unter den Bedingungen des erfindungsgemäßen Verfahrens, d. h. in einer Druckreaktion ohne Abdestillieren einer Reaktionskomponente zur gewünschten Verschiebung des Gleichgewichtes, die Endprodukte in sehr guter Ausbeute und Reinheit liefert.

Das erfindungsgemäße Verfahren weist eine Reihe von Vorteilen auf. Die Durchführung der erfindungsgemäßen Umsetzung erfolgt in einem geschlossenen System, wobei Druck und mögliche hohe Temperaturen zu geringen Reaktionszeiten bei gleich guter Ausbeute führen. Außerdem ist ein geringerer Überschuß an Reduktionsmittel erforderlich. Das erfindungsgemäße Druckverfahren ist somit als sehr wirtschaftlich anzusehen, insbesondere im Hinblick auf eine halbtechnische oder technische Anwendung.

Die nach dem erfindungsgemäßen Verfahren herstellbaren 1-Azolyl-3,3-dimethyl-1-phenoxy-butan-2-ole sind durch die Formel (I) allgemein definiert. In dieser Formel steht Y vorzugsweise für Fluor, Chlor, Brom, Iod, für geradkettiges oder verzweigtes Alkyl mit 1 bis 4 Kohlenstoffatomen, für Alkoxycarbonyl mit 1 oder 2 Kohlenstoffatomen im Alkylteil, für Nitro sowie für gegebenenfalls einfach bis zweifach, gleich oder verschieden substituiertes Phenyl, wobei als Substituenten vorzugsweise genannt seien: Fluor, Chlor, Brom und Methyl. X und der Index n stehen vorzugsweise für die in der Erfindungsdefinition angegebenen Bedeutungen.

Besonders bevorzugt steht $Y_n$ für p-Chlor und p-Phenyl und X für ein Stickstoffatom.

Verwendet man beispielsweise 1-(4-Chlorphenoxy)-3,3-dimethyl-1-(1,2,4-triazol-1-yl)-butan-2-on und Aluminiumisopropylat als Ausgangsstoffe, so kann der Reaktionsablauf durch das folgende Formelschema wiedergegeben werden:

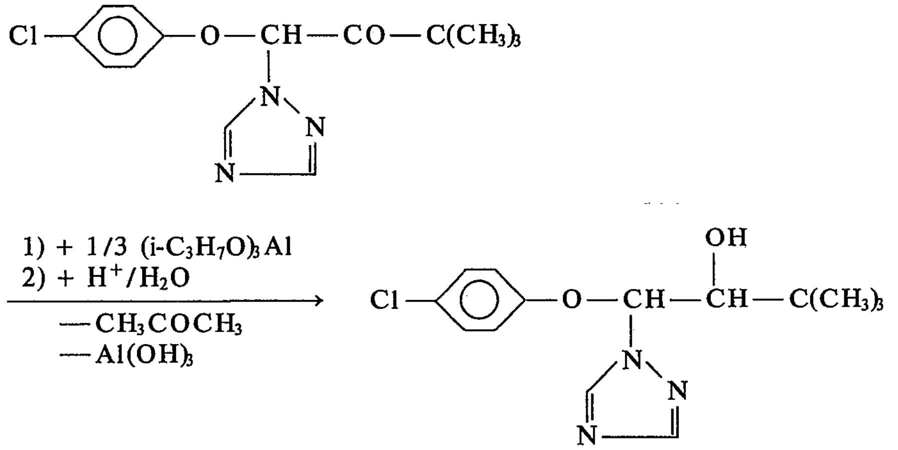


Die bei der Durchführung des erfindungsgemäßen Verfahrens als Ausgangsstoffe zu verwendenden 1-Azolyl-3,3-dimethyl-1-phenoxy-butan-2-one sind durch die Formel (II) allgemein definiert. In dieser Formel stehen X, Y und der Index n vorzugsweise für die Bedeutungen, die bereits im Zusammenhang mit der Beschreibung der erfindungsgemäß herstellbaren Stoffe der Formel (I) vorzugsweise genannt wurden.

Die 1-Azolyl-3,3-dimethyl-1-phenoxy-butan-2-one der Formel (II) sind bekannt (vergleiche DE-PS 2 105 490 [LeA 13 458] und DE-PS 2 201 063 [LeA 14 118]).

Die erfindungsgemäße Reduktion wird mit Hilfe von sekundären Alkoholaten durchgeführt. Hierzu gehören vorzugsweise die sekundären Alkoholate des Aluminiums, wie insbesondere Aluminium-isopropylat und Aluminium-sek.-butylat.

Für die erfindungsgemäße Umsetzung kommen als Verdünnungsmittel vorzugsweise sekundäre Alkohole in Frage, wie insbesondere Isopropanol und sek.-Butanol.

Die erfindungsgemäße Reduktion wird unter Druck durchgeführt, der in einem größeren Bereich variiert werden kann. Im allgemeinen arbeitet man bei einem Überdruck von 1 bis 8 bar, vorzugsweise 1,5 bis 5 bar.

Die Reaktionstemperaturen können beim erfindungsgemäßen Verfahren in einem größeren Bereich variiert werden. Im allgemeinen arbeitet man in einem Temperaturbereich, der 10 bis 60°C, vorzugsweise 20 bis 40°C, oberhalb des Siedepunktes des verwendeten Verdünnungsmittels bei Normaldruck liegt.

Bei der Durchführung der erfindungsgemäßen Umsetzung setzt man auf 1 Mol Keton der Formel (II) vorzugsweise 0,35 bis 0,4 Mol an sekundärem Alkoholat ein. Dabei wird das gesamte Reaktionsgemisch in einem geschlossenen Kessel erhitzt, bis sich die gewünschte Temperatur und der gewünschte Druck eingestellt haben. Nach einer Reaktionszeit von 2 bis 6 Stunden wird das Reaktionsgemisch eingeengt und der Sumpf in üblicher Weise hydrolysiert, wie z. B. mit verdünnter Schwefelsäure und Isopropanol. Die weitere Aufarbeitung erfolgt in üblicher Weise.

Die erfindungsgemäß herstellbaren Stoffe zeichnen sich bekanntermaßen durch sehr gute fungizide Wirksamkeit aus (vergleiche DE-PS 2 324 010 [LeA 14 971], DE-OS 2 333 354 [LeA 15 148] und DE-OS 2 743 767 [LeA 18 424]).

Das erfindungsgemäße Verfahren sei anhand der folgenden Herstellungsbeispiele erläutert.

## Herstellungsbeispiel

### Beispiel 1

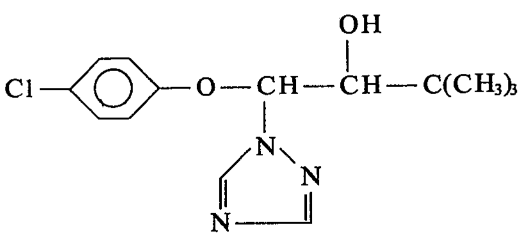

(Halbtechnischer Maßstab)

In einem 3000-l-Rührwerkskessel werden unter Rühren 2000 l Isopropanol, 600 kg 1-(4-Chlorphenoxy)-3,3-dimethyl-1-(1,2,4-triazol-1-yl)-butan-2-on und 200 kg Aluminiumisopropylat gemischt.

Die Mischung wird unter Rühren zum Sieden erhitzt (80° C). Der Kessel wird druckdicht verschlossen und weiter geheizt, bis eine Innentemperatur von 130° C erreicht ist, was einem Kesseldruck von 4 bis 5 bar entspricht. Das Fortschreiten der Reaktion wird gaschromatographisch überprüft, bis vollständige Reduktion erreicht ist. Die Reaktionszeit beträgt 2 bis 3 Stunden. Der Kesselinhalt wird abgekühlt, bis Normaldruck erreicht ist. Der Kessel wird geöffnet. Ein Teil des Lösungsmittels wird abdestilliert (ca. 1000 Liter).

In einem 5000-Liter-Rührkessel wird eine Mischung aus 3000 Liter Wasser und 160 kg konzentrierter Schwefelsäure bei 20° C vorgelegt. Unter Rühren wird der ca. 70° C warme Destillationsrückstand des Reaktionsgemisches langsam zugegeben, wobei das Reaktionsprodukt auskristallisiert.

Nach beendeter Zugabe wird die Suspension auf 10° C gekühlt. Das Kristallisat wird auf einer Rührdrucknutsche abgetrennt, gewaschen und im Vakuum bei 50° C getrocknet. Man erhält 574 kg (95% der Theorie) 1-(4-Chlorphenoxy)-3,3-dimethyl-1-(1,2,4-triazol-1-yl)-butan-2-ol mit einem threo-Anteil von 80% und einem Schmelzbereich von 108—123° C.

Beispiel 2

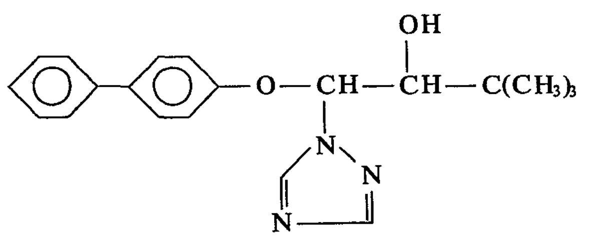

(Halbtechnischer Maßstab)

In einem 3000-l-Rührwerkskessel werden unter Rühren 2000 l Isopropanol, 637 kg 1-(4-Biphenylyloxy)-3,3-dimethyl-1-(1,2,4-triazol-1-yl)-butan-2-on und 200 kg Aluminiumisopropylat gemischt.

Die Mischung wird unter Rühren zum Sieden erhitzt (80° C). Der Kessel wird druckdicht verschlossen und weiter geheizt, bis eine Innentemperatur von 115° C erreicht ist, was einem Kesseldruck von 1,5 bis 2 bar entspricht. Das Fortschreiten der Reaktion wird gaschromatographisch überprüft, bis die vollständige Reduktion erreicht ist. Die Reaktionszeit beträgt 4 bis 6 Stunden. Der Kesselinhalt wird abgekühlt, bis Normaldruck erreicht ist. Der Kessel wird geöffnet.

Ein Teil des Lösungsmittels wird abdestilliert (ca. 1000 Liter).

In einem zweiten 3000-Liter-Rührwerkskessel werden 1050 kg 15prozentiger Schwefelsäure vorgelegt (20° C). Unter Rühren wird der ca. 70° C warme Destillationsrückstand des Reaktionsgemisches zugegeben. Nach Zugabe wird die Mischung eine Stunde bei 60° C gerührt. Bei dieser Verfahrensweise entsteht ein flüssiges Zweiphasengemisch.

Über eine beheizte Scheideflasche werden die Phasen bei 60° C getrennt. Die organische Phase gibt man bei 60° C unter Rühren langsam zu einer Mischung von 2000 Liter Wasser und 45 kg Natronlauge (45%), die bei 20° C in einem 5000-Liter-Rührwerkskessel vorgelegt ist. Hierbei erfolgt die Kristallisation des Reaktionsproduktes. Nach beendeter Zugabe kühlt man den Kesselinhalt auf 10° C und isoliert das kristallisierte Produkt auf einer Rührdrucknutsche. Der Nutschkuchen wird mit Wasser neutral gewaschen und im Vakuum bei 50° C bis zur Gewichtskonstanz getrocknet. Man erhält 609 kg (95% der Theorie) 1-(4-Biphenylyloxy)-3,3-dimethyl-1-(1,2,4-triazol-1-yl)-butan-2-ol mit einem threo-Anteil von 85% und einem Schmelzbereich von 105—131° C.

**Patentansprüche**

1. Verfahren zur Herstellung von 1-Azolyl-3,3-dimethyl-1-phenoxy-butan-2-olen der allgemeinen Formel

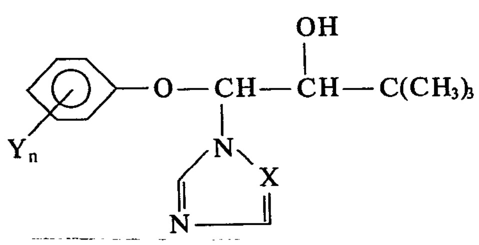

(I)

in welcher

X für ein Stickstoffatom oder die CH-Gruppe steht,
Y für Halogen, Alkyl, Alkoxycarbonyl, Nitro oder gegebenenfalls substituiertes Phenyl steht und
n für ganze Zahlen von 0 bis 3 steht,

dadurch gekennzeichnet, daß man 1-Azolyl-3,3-dimethyl-1-phenoxy-butan-2-one der allgemeinen Formel

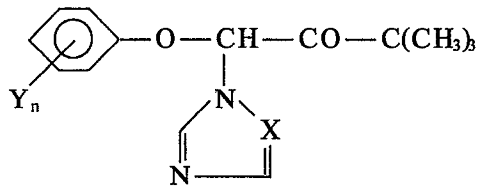
(II)

in welcher
X, Y und n die oben angegebene Bedeutung haben,

in einer Druckreaktion mit sekundären Alkoholaten in Gegenwart eines Verdünnungsmittels umsetzt.

2. Verfahren gemäß Anspruch 1 zur Herstellung von Verbindungen der allgemeinen Formel (I), wobei in Formel (I)

Y für Fluor, Chlor, Brom und Jod, für Alkyl mit 1 bis 4 Kohlenstoffatomen, für Alkoxycarbonyl mit 1 oder 2 Kohlenstoffatomen in Alkylteil, für Nitro und für gegebenenfalls einfach bis zweifach durch Fluor, Chlor, Brom und Methyl substituiertes Phenyl steht,
X für ein Stickstoff oder die CH-Gruppe und
n für ganze Zahlen von 0 bis 3 steht.

3. Verfahren gemäß Anspruch 1 zur Herstellung von Verbindungen der allgemeinen Formel (I), wobei in Formel (I) $Y_n$ für ein p-Chlor-Atom oder eine p-Phenyl-Gruppe und X für ein Stickstoffatom steht.

4. Verfahren gemäß Ansprüchen 1 bis 3, dadurch gekennzeichnet, daß man die Umsetzung mit sekundären Alkoholaten des Aluminiums durchführt.

5. Verfahren gemäß Ansprüchen 1 bis 4, dadurch gekennzeichnet, daß man die Umsetzung mit Aluminiumisopropylat durchführt.

6. Verfahren gemäß Ansprüchen 1 bis 3, dadurch gekennzeichnet, daß man bei einem Überdruck von 1 bis 8 bar arbeitet.

7. Verfahren gemäß Ansprüchen 1 bis 3 und 6, dadurch gekennzeichnet, daß man bei einem Überdruck von 1,5 bis 5 bar arbeitet.

8. Verfahren gemäß Ansprüchen 1 bis 3, dadurch gekennzeichnet, daß man in einem Temperaturbereich arbeitet, der 10 bis 60°C oberhalb des Siedepunktes des verwendeten Lösungsmittels bei Normaldruck liegt.

9. Verfahren gemäß Ansprüchen 1 bis 3 und 8, dadurch gekennzeichnet, daß man in einem Temperaturbereich arbeitet, der 20 bis 40°C oberhalb des Siedepunktes des verwendeten Lösungsmittels liegt.

10. Verfahren gemäß Ansprüchen 1 bis 3, dadurch gekennzeichnet, daß man als Verdünnungsmittel einen sekundären Alkohol verwendet.

## Claims

1. Process for the preparation of 1-azolyl-3,3-dimethyl-1-phenoxybutan-2-ols of the general formula

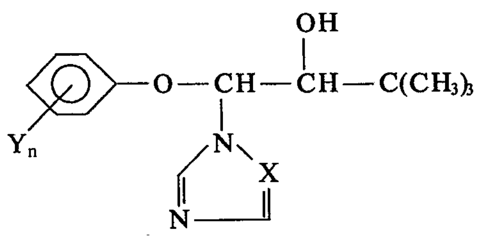

(I)

in which

X represents a nitrogen atom or the CH group,
Y represents halogen, alkyl, alkoxycarbonyl, nitro or optionally substituted phenyl and
n represents integers from 0 to 3,

characterised in that 1-azolyl-3,3-dimethyl-1-phenoxybutan-2-ones of the general formula

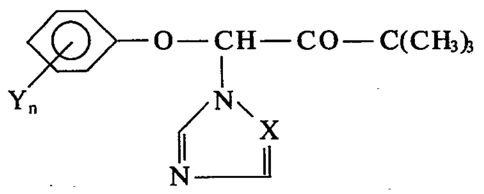

(II)

in which
X, Y and n have the meaning indicated above,

are reacted with secondary alcoholates in a pressure reaction in the presence of a diluent.

2. Process according to Claim 1 for the preparation of compounds of the general formula (I), Y in formula (I) representing fluorine, chlorine, bromine and iodine, alkyl having 1 to 4 carbon atoms, alkoxycarbonyl having 1 or 2 carbon atoms in the alkyl part, nitro and phenyl which is optionally monosubstituted to disubstituted by fluorine, chlorine, bromine and methyl, X represents a nitrogen atom or the CH group and n represents integers from 0 to 3.

3. Process according to Claim 1 for the preparation of compounds of the general formula (I), $Y_n$ in formule (I) representing a p-chlorine atom or a p-phenyl group and X representing a nitrogen atom.

4. Process according to Claims 1 to 3, characterised in that the reaction is carried out using secondary alcoholates of aluminium.

5. Process according to Claims 1 to 4, characterised in that the reaction is carried out using aluminium isopropylate.

6. Process according to Claims 1 to 3, characterised in that the reaction is carried out at an excess pressure of 1 to 8 bar.

7. Process according to Claims 1 to 3 and 6, characterised in that the reaction is carried out at an excess pressure of 1.5 to 5 bar.

8. Process according to Claims 1 to 3, characterised in that the reaction is carried out within a temperature range which is 10 to 60° C above the boiling point, at normal pressure, of the solvent used.

9. Process according to Claims 1 to 3 and 8, characterised in that the reaction is carried out in a temperature range which is 20 to 40° C above the boiling point of the solvent used.

10. Process according to Claims 1 to 3, characterised in that the diluent used is a secondary alcohol.

**Revendications**

1. Procédé de production de 1-azolyl-3,3-diméthyl-1-phénoxybutane-2-ols de formule générale

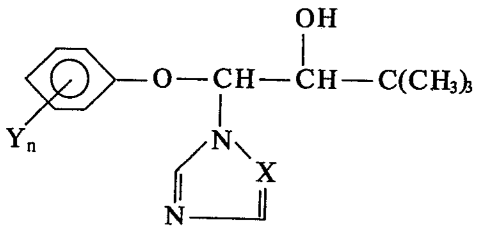

(I)

dans laquelle

X représente un atome d'azote ou le groupe CH,
Y est un halogène, un groupe alkyle, alkoxycarbonyle, nitro ou un groupe phényle éventuellement substitué et
n représente les nombres entiers de 0 à 3,

caractérisé en ce qu'on fait réagir des 1-azolyl-3,3-diméthyl-1-phénoxybutane-2-ones de formule générale

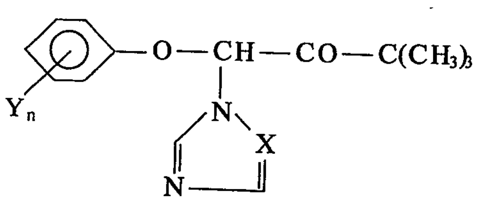

(II)

dans laquelle
X, Y et n ont la définition donnée ci-dessus,

par une réaction sous pression avec des alcoolates secondaires en présence d'un diluant.

2. Procédé suivant la revendication 1 pour la production de composés de formule générale (I), formule (I) dans laquelle

Y représente le fluor, le chlore, le brome et l'iode, un groupe alkyle ayant 1 à 4 atomes de carbone, un groupe alkoxycarbonyle ayant 1 ou 2 atomes de carbone dans la partie alkyle, un groupe nitro ou un groupe phényle éventuellement substitué une ou deux fois par du fluor, du chlore, du brome et un radical méthyle,
X représente l'azote ou le groupe CH et
n représente des nombres entiers de 0 à 3.

3. Procédé suivant la revendication 1 pour l'obtention de composés de formule générale (I), formule (I) dans laquelle $Y_n$ est un atome de chlore en position para ou un groupe phényle en position para et X est un atome d'azote.

4. Procédé suivant les revendications 1 à 3, caractérisé en ce qu'on conduit la réaction avec des alcoolates secondaires de l'aluminium.

Procédé suivant les revendications 1 à 4, caractérisé en ce qu'on conduit la réaction avec l'isopropylate d'aluminium.

6. Procédé suivant les revendications 1 à 3, caractérisé en ce qu'on opère avec une surpression de 1 à 8 bars.

7. Procédé suivant les revendications 1 à 3 et 6, caractérisé en ce qu'on opère à une surpression de 1,5 à 5 bars.

8. Procédé suivant les revendications 1 à 3, caractérisé en ce qu'on opère dans un intervalle de température qui se situe à 10—60° C au-dessus du point d'ébullition à la pression normale du solvant utilisé.

9. Procédé suivant les revendications 1 à 3 et 8, caractérisé en ce qu'on opère dans un intervalle de température qui se situe à 20—40° C au-dessus du point d'ébullition du solvant utilisé.

10. Procédé suivant les revendications 1 à 3, caractérisé en ce qu'on utilise comme diluant un alcool secondaire.